# EUROPÄISCHE PATENTSCHRIFT

(11) **EP 1 276 841 B1**
(45) Veröffentlichungstag und Bekanntmachung des Hinweises auf die Patenterteilung: **10.03.2004**
(21) Anmeldenummer: 01927871.2
(22) Anmeldetag: 04.04.2001
(51) Int. Cl.: C11D 11/00, C08G 65/26

(54) **ALKOHOLALKOXYLATE ALS SCHAUMARME ODER SCHAUMDÄMPFENDE TENSIDE**
ALCOHOL ALCOXYLATES USED AS LOW-FOAM, OR FOAM-INHIBITING SURFACTANTS
ALCOXYLATES D'ALCOOL UTILISES COMME TENSIOACTIFS A FAIBLE PRODUCTION DE MOUSSE OU A EFFET ANTIMOUSSE

(30) Priorität: 07.04.2000 DE 10017197
(43) Veröffentlichungstag der Anmeldung: 22.01.2003
(73) Patentinhaber: BASF AKTIENGESELLSCHAFT, 67056 Ludwigshafen (DE)
(72) Erfinder: GÜMBEL, Helmut, 67814 Dannenfels (DE); AUS DEM KAHMEN, Martin, 67071 Ludwigshafen (DE); WAGNER, Norbert, 67112 Mutterstadt (DE); TAEGER, Klaus, 67251 Freinsheim (DE)
(74) Vertreter: Isenbruck, Günter, Dr.
(86) Internationale Anmeldenummer: PCT/EP2001/003850
(87) Internationale Veröffentlichungsnummer: WO 2001/077276

(56) Entgegenhaltungen:
- EP-A- 0 681 865
- WO-A-96/12001
- US-A- 4 827 028
- US-A- 5 294 365
- US-A- 5 766 371

## Beschreibung

Die Erfindung betrifft Alkoholalkoxylate und deren Gemische, die als schaumarme oder schaumdämpfende Tenside beispielsweise in Wasch- und Reinigungsmitteln und in Formulierungen für chemisch-technische Anwendungen eingesetzt werden können, sowie diese enthaltende Wasch- und Reinigungsmittel.

Schaumarme oder schaumdämpfende Tenside aus ethoxylierten und propoxylierten Alkoholen sind an sich bekannt. US 5,766,371 betrifft bioabbaubare schaumarme Tenside, die als Klarspüler in Geschirrspülmitteln verwendet werden können. Es werden zunächst mit Propylenoxid und sodann mit Ethylenoxid umgesetzte C₄₋₁₈-Alkanole beschrieben, die endständig mit einem C₄₋₈-Alkylenoxid umgesetzt sind.

US 5,294,365 betrifft Hydroxypolyether, die als schaumarme Tenside eingesetzt werden können. Dabei werden C₁₋₁₈-Alkohole zunächst mit Ethylenoxid, sodann mit Propylenoxid und anschließend mit Glycidylethern umgesetzt.

US 4,827,028 betrifft anionische Tenside, die durch Umsetzung von C₁₋₈-Alkoholen mit Ethylenoxid, sodann Propylenoxid und anschließend einem Alkylenoxid mit mindestens 8 Kohlenstoffatomen erhalten werden. Die Reihenfolge der Umsetzung mit Propylenoxid und Ethylenoxid kann zudem umgekehrt werden.

WO 96/12001 betrifft bioabbaubare Tenside, die als Klarspüler verwendet werden können. Die Tenside werden überwiegend durch Umsetzung von C₄₋₁₈-Alkoholen mit Propylenoxid, sodann Ethylenoxid und anschließend C₄₋₁₈-Alkylenoxiden erhalten. In den Beispielen wird auch die Umsetzung von C₆₋₁₀-Alkoholen mit mindestens 20 mol Ethylenoxid und anschließend Butylenoxid oder Decylenoxid beschrieben.

EP 681 865 betrifft schaumarme Netzmittel und ihre Verwendung. Das Netzmittel weist 30-90 Gew.% Alkohol ethoxylate oder kombinierte Ethoxylate/Propoxylate und 10-70 Gew.% Alkohol propoxylate auf. Die Alkohol komponente enthält dabei 4-20 Kohlenstoffatome.

Die bekannten schaumarmen Tenside weisen nicht für alle Anwendungen eine geeignete Eigenschaftskombination aus Schaumdämpfung, Netzwirkung und Formulierbarkeit auf.

Mitunter weisen die bekannten Tenside auch ein relativ hohes ökotoxikologisches Gefährdungspotential auf, insbesondere gegenüber aquatischen Organismen.

Aufgabe der vorliegenden Erfindung ist daher die Bereitstellung von Alkoholalkoxylaten, die ein verbessertes Eigenschaftsspektrum aufweisen und insbesondere als schaumarme und schaumdämpfende Tenside eingesetzt werden können. Dabei sollen sie sich insbesondere für den Einsatz in Wasch- und Reinigungsmitteln und für chemisch-technische Anwendungen eignen.

Die Aufgabe wird erfindungsgemäß gelöst durch Alkoholalkoxylate der allgemeinen Formel (I)

R¹-O-(CH₂-CHR⁵-O-)ᵣ(CH₂-CH₂-O-)ₙ(CH₂-CHR⁶-O-)ₛ(CH₂-CHR²-O-)ₘH (I)

mit der Bedeutung
- R¹: mindestens einfach verzweigtes C₄₋₂₂-Alkyl,
- R²: C₃₋₄-Alkyl
- R⁵: C₁₋₄-Alkyl
- R⁶: Methyl oder Ethyl
- n: mittlerer Wert von 1 bis 50
- m: mittlerer Wert von 0 bis 20, vorzugsweise 0,5 bis 20
- r: mittlerer Wert von 0 bis 50
- s: mittlerer Wert von 0 bis 50,
wobei m mindestens 0,5 ist, wenn R⁵ Methyl oder Ethyl ist oder r den Wert 0 hat.

Die Aufgabe wird zudem erfindungsgemäß gelöst durch ein Gemisch aus 20 bis 95, vorzugsweise 30 bis 95 Gew.-% mindestens eines vorstehenden Alkoholalkoxylats und 5 bis 80, vorzugsweise 5 bis 70 Gew.-% eines entsprechenden Alkoholalkoxylats, in dem R¹ jedoch ein unverzweigter Alkylrest mit gleicher Kohlenstoffzahl ist.

Die Aufgabe wird ferner gelöst durch Alkoholalkoxylate der allgemeinen Formel (II)

R³-O-(CH₂-CH₂-O)ₚ(CH₂-CHR⁴-O-)_{q}H (II)

mit der Bedeutung
- R³: verzweigtes oder unverzweigtes C₄₋₂₂-Alkyl
- R⁴: C₃₋₄-Alkyl
- p: mittlerer Wert von 1 bis 50, vorzugsweise 4 bis 15
- q: mittlerer Wert von 0,5 bis 20, bevorzugt 0,5 bis 4, bevorzugter 0,5 bis 2

Die Aufgabe wird zudem gelöst durch Gemische aus 5 bis 95 Gew.-% mindestens eines verzweigten Alkoholalkoxylats (II), wie es unmittelbar vorstehend beschrieben ist, und 5 bis 95 Gew.-% eines entsprechenden Alkoholalkoxylats, in dem anstelle eines verzweigten Alkylrestes jedoch ein unverzweigter Alkylrest vorliegt.

In den Alkoholalkoxylaten der allgemeinen Formel (I) ist R² vorzugsweise Propyl, insbesondere n-Propyl.

Vorzugsweise weist in den Alkoholalkoxylaten der allgemeinen Formel (I) n einen mittleren Wert von 4 bis 15, besonders bevorzugt 6 bis 12, insbesondere 7 bis 10 auf.

Bevorzugt weist m einen mittleren Wert von 0,5 bis 4, besonders bevorzugt 0,5 bis 2, insbesondere 1 bis 2 auf. Der Ausdruck "mittlerer Wert" bezieht sich auf technische Produkte, in denen in den Einzelmolekülen unterschiedliche Anzahlen an Alkylenoxideinheiten vorliegen können. Er beschreibt den in technischen Produkten im Mittel vorliegenden Anteil der entsprechenden AlkylenoxidEinheiten. Ein Wert von 0,5 bedeutet daher, daß im Mittel jedes zweite Molekül eine entsprechende Einheit trägt. Anstelle der Untergrenze von 0,5 tritt gemäß einer bevorzugten Ausführungsform der Erfindung für die Indices n, m, p, q die Untergrenze 1.

r ist vorzugsweise 0. s ist vorzugsweise 0.

Der Rest R¹ ist vorzugsweise C₈₋₁₅-, besonders bevorzugt C₈₋₁₃-, insbesondere C₈₋₁₂-Alkylrest, der mindestens einfach verzweigt ist. Es können auch mehrere Verzweigungen vorliegen.

R⁵ ist vorzugsweise Methyl oder Ethyl, insbesondere Methyl.
R⁶ ist vorzugsweise Ethyl.

In den Gemischen liegen Verbindungen mit unverzweigten und mit verzweigten Alkoholresten R' vor. Dies ist beispielsweise der Fall bei Oxoalkoholen, die einen Anteil an linearen und einen Anteil an verzweigten Alkoholketten aufweisen. Beispielsweise weist ein C_{13/15}-Oxoalkohol häufig etwa 60 Gew.-% vollständig linearer Alkoholketten, daneben aber auch etwa 40 Gew.-% α-Methyl-verzweigte und C_{≥2}-verzweigte Alkoholketten auf.

In den Alkoholalkoxylaten der allgemeinen Formel (II) ist R³ vorzugsweise ein verzweigter oder unverzweigter C₈₋₁₅-Alkylrest, besonders bevorzugt ein verzweigter oder unverzweigter C₈₋₁₃-Alkylrest und insbesondere ein verzweigter oder unverzweigter C₈₋₁₂-Alkylrest. R⁴ ist vorzugsweise Propyl, insbesondere n-Propyl. p weist vorzugsweise einen mittleren Wert von 4 bis 15, besonders bevorzugt einen mittleren Wert von 6 bis 12 und insbesondere einen mittleren Wert von 7 bis 10 auf. q weist vorzugsweise einen mittleren Wert von 0,5 bis 4, besonders bevorzugt 0,5 bis 2, insbesondere 1 bis 2 auf.

Entsprechend den Alkoholalkoxylaten der allgemeinen Formel (I) können auch die Alkoholalkoxylate der allgemeinen Formel (II) als Gemische mit unverzweigten und verzweigten Alkoholresten vorliegen.

Als den erfindungsgemäßen Alkoholalkoxylaten zugrunde liegende Alkoholkomponenten kommen nicht nur reine Alkanole in Betracht, sondern auch homologe Mischungen mit einem Bereich von Kohlenstoffatomen. Beispiele sind C_{8/10}-Alkanole, C_{10/12}-Alkanole, C_{13/15}-Alkanole, C_{12/15}-Alkanole. Auch Gemische mehrerer Alkanole sind möglich.

Die vorstehenden erfindungsgemäßen Alkanolalkoxylate oder Gemische werden vorzugsweise hergestellt durch Umsetzung von Alkoholen der allgemeinen Formel R¹-OH bzw. R³-OH oder Gemischen entsprechender verzweigter und unverzweigter Alkohole, gegebenenfalls zuerst mit C₃₋₆-Alkylenoxid, sodann mit Ethylenoxid und nachfolgend gegebenenfalls mit C₃₋₄-Alkylenoxid und sodann einem entsprechenden C₅₋₆-Alkylenoxid. Die Alkoxylierungen werden dabei vorzugsweise in Gegenwart von Alkoxylierungskatalysatoren durchgeführt. Dabei werden insbesondere basische Katalysatoren wie Kaliumhydroxid eingesetzt. Durch spezielle Alkoxylierungskatalysatoren wie modifizierte Bentonite oder Hydrotalcite, wie sie beispielsweise in WO 95/04024 beschrieben sind, kann die statistische Verteilung der Mengen der eingebauten Alkylenoxide stark eingeengt werden, so daß man "Narrow-Range"-Alkoxylate erhält. Dadurch kann die statistische Verteilung der Menge an Alkylenoxideinheiten in technischen Gemischen stark eingeschränkt werden.

Die erfindungsgemäßen Alkoholalkoxylate oder deren Gemische werden erfindungsgemäß bevorzugt als schaumarme oder schaumdämpfende Tenside eingesetzt.

Die erfindungsgemäßen schaumarmen oder schaumdämpfenden Tenside können dabei in einer Vielzahl von Anwendungen eingesetzt werden. Vorzugsweise werden sie als nichtionische Tenside vorzugsweise in Wasch- und Reinigungsmittel-Formulierungen und in tensidhaltigen Formulierungen für chemisch-technische Anwendungen eingesetzt, beispielsweise für Reinigungsprozesse in Technik und Haushalt wie für die Textilwäsche oder für Reinigungsprozesse im Nahrungsmittelbereich wie die Reinigung von Getränkeflaschen oder von Behältern oder Anlagen in der nahrungsmittelverarbeitenden Industrie oder in Geschirreinigungsmitteln. Insbesondere ist hier die Reinigung harter Oberflächen aus beispielsweise Glas, Keramik, Lack, Kunststoff oder Metall von Interesse. Die Tenside finden weiterhin Anwendung in technischen Reinigern und in Reinigungsprozessen in der metallverarbeitenden Industrie.

Die Tenside können auch mit Vorteil für eine Vielzahl anderer chemischtechnischer Prozesse eingesetzt werden, so generell in der metallverarbeitenden Industrie, beispielsweise in
- Kühlschmierstoffen,
- Härteölen,
- Hydraulikölemulsionen,
- Polierpasten,
- Formtrennmitteln,
- Ziehölen,
- Beizmitteln,
- Metallreinigern,
- Metalltrocknem.

Hierbei können Tenside speziell in den Prozessen im Vorteil eingesetzt werden, in denen es auf eine hohe thermische Stabilität ankommt.

Weiterhin können die Tenside bei der Herstellung und Verarbeitung von Textilien eingesetzt werden. Die Anwendung von Tensiden bei der Herstellung und Verarbeitung von Textilien ist außerordentlich vielseitig, sie erstreckt sich hauptsächlich auf die Gebiete
- Vorbehandlungsmittel von Fasern,
- Herstellung von Reyon-Fasern,
- Spinnpräparationen und Textilschmälzen,
- Färbehilfsmittel,
- Avivagen,
- Hydrophobiermittel,
- Hilfsmittel für den Druck,
- Antistatika,
- Beflockungs- und Beschichtungsmittel.

Weiterhin können Tenside in der Leder-, Papier-, Druck-, Galvano- und Photoindustrie verwendet werden. Wichtige Anwendungsgebiete hierbei sind Lacke, Pigmente und Druckfarben. Eingesetzt werden Tenside in diesen Anwendungsgebieten sowohl in wäßrigen als auch in nichtwäßrigen Systemen. In nichtwäßrigen Systemen dienen sie vor allem als Dispergierhilfsmittel, Antiabsetzmittel oder Verlaufshilfsmittel. Zudem ermöglichen Tenside die Herstellung von sogenannten High-Solids-Systemen. Einen größeren Anteil haben Tenside in wäßrigen Systemen, in denen sie neben der Stabilisierung der durch Emulsionspolymerisation oder -polykondensation hergestellten Bindemittel auf Kunststoffdispersionsbasis auch als Dispergierhilfsmittel oftmals eingesetzter organischer und anorganischer Pigmente dienen. Daneben verbessern sie die Hafteigenschaften dieser Anstrichmittel.

Weiterhin können die Tenside bei der Wasserbehandlung, beispielsweise bei der Abwasserreinigung, verwendet werden.

Weiterhin können die Tenside in Pflanzenschutzformulierungen eingesetzt werden.

Weiterhin können die Verbindungen als Tenside oder Emulgatoren in der kunststoffherstellenden und kunststoffverarbeitenden Industrie verwendet werden.

Hauptanwendungsgebiete bei der Kunststoffherstellung und -verarbeitung sind
- Herstellung von Kunststoffdispersionen,
- Herstellung von Perlpolymerisaten,
- Herstellung von Schaumstoffen,
- Verwendung von grenzflächenaktiven Formtrennmitteln,
- Herstellung von Mikrokapseln
- Verbesserung der Adhäsion zwischen Füll- und Kunststoffen,
- Zusätze zu Kunststoffdispersionen zur Erzielung besonderer Effekte wie Verschäumbarkeit, Füllstoffverträglichkeit oder Netzvermögen,
- Emulgatoren für nichtwäßrige Systeme,
- Anfärben von Kunststoffen
- Antistatische Ausrüstung von Kunststoffen,
- Klebstoffe.

Gegenstand der vorliegenden Erfindung sind ebenfalls Wasch- und Reinigungsmittel-Formulierungen, welche (neben den üblichen Bestandteilen) als Tenside 0,1 bis 40 Gew.-%, insbesondere 1 bis 30 Gew.-%, vor allem 10 bis 25 Gew.-%, bezogen auf die Gesamtmenge der Formulierung, mindestens einer erfindungsgemäßen Verbindung der Formeln (I) oder (II) enthalten. Daneben können diese Formulierungen noch weitere nichtionische Tenside, aber auch kationische, anionische und/oder amphotere Tenside enthalten. Die üblichen Bestandteile von Wasch- und Reinigungsmittel-Formulierungen sind dem Fachmann bekannt.

Die beschriebenen Alkoxylate zeigen meist eine ungewöhnlich niedrige Grenzflächenspannung, vor allem gegen unpolare Öle wie Motorenöle (bei Anwendung in technischen Reinigern von Bedeutung), was sehr gut mit einem hohen Fettablösevermögen korreliert, eine wirksame Erniedrigung der Oberflächenspannung bewirkt sowie eine sehr niedrige kritische Micellbildungskonzentration zur Folge hat. Sie zeigen in der Regel ein sehr gutes Benetzungsvermögen harter Oberflächen und ein sehr gutes Netzvermögen auf textilen Oberflächen. Sie erzeugen beim Waschvorgang meist wenig Schaum, was sie für Textilwaschmittel, insbesondere Pulverwaschmittel, geeignet macht. Sie erzeugen auch in maschinellen und manuellen Reinigungsprozessen in der Regel nur wenig Schaum, wirken meist schaumdämpfend und bewirken meist einen raschen Schaumzerfall.

Die beschriebenen Alkoxylate sind leicht biologisch abbaubar und toxikologisch weitgehend unbedenklich, insbesondere ist ihre aquatische Toxizität wesentlich geringer als bei vergleichbaren marktüblichen Produkten.

Die Erfindung wird durch die nachstehenden Beispiele näher erläutert.

### Beispiel 1: i-Decanol + 10 EO + 1,5 Pentenoxid

In einem Autoklaven wurden 474 g i-Decanol (entsprechend 3,0 mol) zusammen mit 4,5 g Kaliumhydroxid als Alkoxylierungskatalysator vorgelegt. Nach einer Entwässerungsphase wurden bei 110 bis 120°C kontinuierlich 1320 g Ethylenoxid (entsprechend 30,0 mol) eingegast. Zur Vervollständigung der Umsetzung wurde 1 h bei derselben Temperatur nachgerührt. Dann wurde die Temperatur auf 150 °C erhöht, und es wurden kontinuierlich 387 g Pentenoxid (entsprechend 4,5 mol) in den Reaktor gegeben. Bei Erreichen der Druckkonstanz wurde die Temperatur zur Vervollständigung des Umsatzes zwei Stunden gehalten. Man erhielt 2180 g des oben genannten Produktes.

Die Substanzen der Beispiele 2 bis 5 wurden analog hergestellt.

### Beispiel Nr. 2: i-Decanol + 10 EO + 1 Pentenoxid

### Beispiel Nr. 3: i-Decanol + 10 EO + 2 Pentenoxid

### Beispiel Nr. 4: i-Decanol + 7,3 EO + 1,5 Pentenoxid

### Beispiel Nr. 5: i-Decanol + 15 EO + 1,5 Pentenoxid

### Beispiel Nr. 6 C_{13/15}-Oxoalkohol + 10 EO + 2 Pentenoxid

In einem Autoklaven wurden 424 g C_{13/15}-Oxoalkohol (entsprechend 2,0 mol) zusammen mit 4,0 g Kaliumhydroxid als Alkoxylierungskatalysator vorgelegt. Nach einer Entwässerungsphase wurden bei 110 bis 120 °C kontinuierlich 880 g Ethylenoxid (entsprechend 20,0 mol) eingegast. Zur Vervollständigung der Umsetzung wurde 1 h bei derselben Temperatur nachgerührt. Dann wurde die Temperatur auf 155 °C erhöht, und es wurden kontinuierlich 344 g Pentenoxid (entsprechend 4 mol) in den Reaktor gegeben. Bei Erreichen der Druckkonstanz wurde die Temperatur zur Vervollständigung des Umsatzes zwei Stunden gehalten. Man erhielt 1642 g des oben genannten Produktes.

### Beispiel 7: C_{8/10}-Alkohol + 10 EO + 2 Pentenoxid

In einem Autoklaven wurden 486 g C_{8/10}-Alkohol (entsprechend 3,0 mol) zusammen mit 5,0 g Kaliumhydroxid als Alkoxylierungskatalysator vorgelegt. Nach einer Entwässerungsphase wurden bei 110 bis 120 °C kontinuierlich 1320 g Ethylenoxid (entsprechend 30,0 mol) eingegast. Zur Vervollständigung der Umsetzung wurde 1 h bei derselben Temperatur nachgerührt. Dann wurde die Temperatur auf 150 °C erhöht, und es wurden kontinuierlich 516 g Pentenoxid (entsprechend 6 mol) in den Reaktor gegeben. Bei Erreichen der Druckkonstanz wurde die Temperatur zur Vervollständigung des Umsatzes zwei Stunden gehalten. Man erhielt 2309 g des oben genannten Produkts.

### Beispiel 8: 2-Ethylhexanol + 10 EO + 2 Pentenoxid

In einem Autoklaven wurden 390 g 2-Ethylhexanol (entsprechend 3,0 mol) zusammen mit 5,0 g Kaliumhydroxid als Alkoxylierungskatalysator vorgelegt. Nach einer Entwässerungsphase wurden bei 110 bis 120 °C kontinuierlich 1320 g Ethylenoxid (entsprechend 30,0 mol) eingegast. Zur Vervollständigung der Umsetzung wurde 1 h bei derselben Temperatur nachgerührt. Dann wurde die Temperatur auf 150°C erhöht, und es wurden kontinuierlich 516 g Pentenoxid (entsprechend 6 mol) in den Reaktor gegeben. Bei Erreichen der Druckkonstanz wurde die Temperatur zur Vervollständigung des Umsatzes zwei Stunden gehalten. Man erhielt 2214 g des oben genannten Produkts.

### Beispiel 9: 2-Propylheptanol + 10 EO + 1,5 Pentenoxid

In einem Autoklaven wurden 316 g 2-Propylheptanol (entsprechend 2,0 mol) zusammen mit 4,0 g Kaliumhydroxid als Alkoxylierungskatalysator vorgelegt. Nach einer Entwässerungsphase wurden bei 110 bis 120 °C kontinuierlich 880 g Ethylenoxid (entsprechend 20,0 mol) eingegast. Zur Vervollständigung der Umsetzung wurde 1 h bei derselben Temperatur nachgerührt. Dann wurde die Temperatur auf 155 °C erhöht, und es wurden kontinuierlich 258 g Pentenoxid (entsprechend 3 mol) in den Reaktor gegeben. Bei Erreichen der Druckkonstanz wurde die Temperatur zur Vervollständigung des Umsatzes zwei Stunden gehalten. Man erhielt 1440 g des oben genannten Produkts.

### Beispiel 10: C_{10/12}-Alkohol + 7,6 EO + 1,5 Pentenoxid

In einem Autoklaven wurden 81 g C_{10/12}-Alkohol (entsprechend 0,5 mol) zusammen mit 1,56 g Kaliumhydroxid als Alkoxylierungskatalysator vorgelegt. Nach einer Entwässerungsphase wurden bei 110 bis 120°C kontinuierlich 167,2 g Ethylenoxid (entsprechend 3,8 mol) eingegast. Zur Vervollständigung der Umsetzung wurde 1 h bei derselben Temperatur nachgerührt. Dann wurden bei 120 °C kontinuierlich 129 g Pentenoxid (entsprechend 1,5 mol) in den Reaktor gegeben und die Temperatur auf 150 °C erhöht. Bei Erreichen der Druckkonstanz wurde die Temperatur zur Vervollständigung des Umsatzes 2 Stunden gehalten. Man erhielt 310 g der Substanz des Beispiels 10.

Die Substanzen der Beispiele 11 und 12 wurden analog hergestellt.

### Beispiel 11: C_{10/12}-Alkohol + 10 EO + 1,5 Pentenoxid

### Beispiel 12: C_{10/12}-Alkohol + 10 EO + 2 Pentenoxid

### Beispiel 13: n-Butyldiglykol + 10 EO + 6 Pentenoxid

In einem Autoklaven wurden 81 g n-Butyldiglykol (entsprechend 0,5 mol) zusammen mit 1,5 g Kaliumhydroxid als Alkoxylierungskatalysator vorgelegt. Nach einer Entwässerungsphase wurden bei 110 bis 120°C kontinuierlich 220 g Ethylenoxid (entsprechend 5,0 mol) eingegast. Zur Vervollständigung der Umsetzung wurde 1 h bei derselben Temperatur nachgerührt. Dann wurde bei 120°C kontinuierlich 258 g Pentenoxid (entsprechend 3,0 mol) in den Reaktor gegeben und die Temperatur auf 150 °C erhöht. Bei Erreichen der Druckkonstanz wurde die Temperatur zur Vervollständigung des Umsatzes 2 Stunden gehalten. Man erhielt 560 g der Substanz des Beispiels 13.

### Beispiel 14: n-Hexylglykol + 12 EO + 4 Pentenoxid

In einem Autoklaven wurden 73 g n-Hexylglykol (entsprechend 0,5 mol) zusammen mit 1,3 g Kaliumhydroxid als Alkoxylierungskatalysator vorgelegt. Nach einer Entwässerungsphase wurden bei 110 bis 120°C kontinuierlich 264 g Ethylenoxid (entsprechend 6,0 mol) eingegast. Zur Vervollständigung der Umsetzung wurde 1 h bei derselben Temperatur nachgerührt. Dann wurden bei 120 °C kontinuierlich 172 g Pentenoxid (entsprechend 2,0 mol) in den Reaktor gegeben und die Temperatur auf 150 °C erhöht. Bei Erreichen der Druckkonstanz wurde die Temperatur zur Vervollständigung des Umsatzes 2 Stunden gehalten. Man erhielt 510 g der Substanz des Beispiels 14.

### Beispiel 15: C₁₁-Alkohol + 10 EO + 2 Pentenoxid

In einem Autoklaven wurden 344 g C₁₁-Alkohol (entsprechend 2,0 mol) zusammen mit 3,1 g Kaliumhydroxid als Alkoxylierungskatalysator vorgelegt. Nach einer Entwässerungsphase wurden bei 110 bis 120°C kontinuierlich 880 g Ethylenoxid (entsprechend 20,0 mol) eingegast. Zur Vervollständigung der Umsetzung wurde 1 h bei derselben Temperatur nachgerührt. Dann wurde die Temperatur auf 150°C erhöht und kontinuierlich 344 g Pentenoxid (entsprechend 4,0 mol) in den Reaktor gegeben. Bei Erreichen der Druckkonstanz wurde die Temperatur zur Vervollständigung des Umsatzes zwei Stunden gehalten. Man erhielt 1541 g der Substanz des Beispiels 15.

Die Substanz des Beispiels 16 wurde analog hergestellt.

### Beispiel 16: C_{12/15}-Alkohol + 12 EO + 2 Pentenoxid

### Beispiel 17: 2-Butyloctanol + 10 EO + 1,5 Pentenoxid

In einem Autoklaven wurden 372 g 2-Butyloctanol (entsprechend 2,0 mol) zusammen mit 3,8 g Kaliumhydroxid als Alkoxylierungskatalysator vorgelegt. Nach einer Entwässerungsphase wurden bei 110 bis 120°C kontinuierlich 880 g Ethylenoxid (entsprechend 20,0 mol) eingegast. Zur Vervollständigung der Umsetzung wurde 1 h bei derselben Temperatur nachgerührt. Dann wurde die Temperatur auf 150°C erhöht und kontinuierlich 258 g Pentenoxid (entsprechend 3,0 mol) in den Reaktor gegeben. Bei Erreichen der Druckkonstanz wurde die Temperatur zur Vervollständigung des Umsatzes zwei Stunden gehalten. Man erhielt 1510 g der Substanz des Beispiels 17.

Die Substanzen der Beispiele 18 und 19 wurden analog hergestellt.

### Beispiel 18: 2-Hexyldecanol + 11 EO + 2 Pentenoxid

### Beispiel 19: 2-Ochyldodecanol + 12 EO + 2 Pentenoxid

### Beispiel 20: i-Decanol + 1,5 Pentenoxid + 6 EO + 3 PO

In einem Autoklaven wurden 237 g i-Decanol (entsprechend 1,5 mol) zusammen mit 2,7 g Kaliumhydroxid als Alkoxylierungskatalysator vorgelegt. Nach einer Entwässerungsphase wurden 193,5 g Pentenoxid (entsprechend 2,25 mol) kontinuierlich in den Reaktor gegeben und die Temperatur auf 150 °C erhöht. Zur Vervollständigung der Umsetzung wurde 2 h bei derselben Temperatur gehalten. Dann wurde die Temperatur auf 120 bis 130 °C gesenkt und kontinuierlich 396 g Ethylenoxid (entsprechend 9,0 mol) eingegast. Zur Vervollständigung der Umsetzung wurde erneut 1 h nachgerührt. Danach wurden bei gleicher Temperatur kontinuierlich 261,5 g Propylenoxid (entsprechend 4,5 mol) eingegast. Bei Erreichen der Druckkonstanz wurde die Temperatur zur Vervollständigung des Umsatzes 1,5 h gehalten. Man erhielt 1088 g der Substanz des Beispiels 20.

### Beispiel 21: i-Decanol + 1,5 Pentenoxid + 8 EO + 2 BuO

In einem Autoklaven wurden 237 g i-Decanol (entsprechend 1,5 mol) zusammen mit 2,9 g Kaliumhydroxid als Alkoxylierungskatalysator vorgelegt. Nach einer Entwässerungsphase wurden 193,5 g Pentenoxid (entsprechend 2,25 mol) kontinuierlich in den Reaktor gegeben und die Temperatur auf 150 °C erhöht. Zur Vervollständigung der Umsetzung wurde 2 h bei derselben Temperatur gehalten. Dann wurde die Temperatur auf 120 bis 130 °C gesenkt und kontinuierlich 528 g Ethylenoxid (entsprechend 12,0 mol) eingegast. Zur Vervollständigung der Umsetzung wurde erneut 1 h nachgerührt. Danach wurden die Temperatur auf 130 bis 140 °C erhöht und kontinuierlich 216 g Butylenoxid (entsprechend 3,0 mol) eingegast. Bei Erreichen der Druckkonstanz wurde die Temperatur zur Vervollständigung des Umsatzes 1,5 h gehalten. Man erhielt 1174 g der Substanz des Beispiels 21.

### Beispiel 22: i-Decanol + 1 PO + 12 EO + 1,5 Pentenoxid

In einem Autoklaven wurden 158 g i-Decanol (entsprechend 1,0 mol) zusammen mit 2,2 g Kaliumhydroxid als Alkoxylierungskatalysator vorgelegt. Nach einer Entwässerungsphase wurden bei 120 bis 130 °C kontinuierlich 58 g Propylenoxid (entsprechend 1,0 mol) eingegast. Zur Vervollständigung der Umsetzung wurde 1,5 h bei der selben Temperatur nachgerührt. Danach wurden bei gleicher Temperatur kontinuierlich 528 g Ethylenoxid (entsprechend 12,0 mol) eingegast und zur Vervollständigung der Umsetzung nochmals 1 h bei gleicher Temperatur gehalten. Dann wurden bei 130 °C kontinuierlich 129 g Pentenoxid (entsprechend 1,5 mol) in den Reaktor gegeben und die Temperatur auf 150 °C erhöht. Bei Erreichen der Druckkonstanz wurde die Temperatur zur Vervollständigung des Umsatzes 2 Stunden gehalten. Man erhielt 870 g der Substanz des Beispiels 22.

### Beispiel 23: i-Decanol + 12 EO + 1 PO + 1,5 Pentenoxid

In einem Autoklaven wurden 158 g i-Decanol (entsprechend 1,0 mol) zusammen mit 2,2 g Kaliumhydroxid als Alkoxylierungskatalysator vorgelegt. Nach einer Entwässerungsphase wurden bei 110 bis 120 °C kontinuierlich 528 g Ethylenoxid (entsprechend 12,0 mol) eingegast. Zur Vervollständigung der Umsetzung wurde 1 h bei der selben Temperatur nachgerührt. Danach wurde die Temperatur auf 130 °C erhöht und kontinuierlich 58 g Propylenoxid (entsprechend 1,0 mol) eingegast und zur Vervollständigung der Umsetzung nochmals 1,5 h bei gleicher Temperatur gehalten. Dann wurden bei 130 °C kontinuierlich 129 g Pentenoxid (entsprechend 1,5 mol) in den Reaktor gegeben und die Temperatur auf 150 °C erhöht. Bei Erreichen der Druckkonstanz wurde die Temperatur zur Vervollständigung des Umsatzes zwei Stunden gehalten. Man erhielt 874 g der Substanz des Beispiels 23.

### Beispiel 24: i-Decanol + 14 EO + 2 Hexenoxid

In einem Autoklaven wurden 79 g i-Decanol (entsprechend 0,5 mol) zusammen mit 1,2 g Kaliumhydroxid als Alkoxylierungskatalysator vorgelegt. Nach einer Entwässerungsphase wurden bei 110 bis 120°C kontinuierlich 308 g Ethylenoxid (entsprechend 7,0 mol) eingegast. Zur Vervollständigung der Umsetzung wurde 1 h bei derselben Temperatur nachgerührt. Dann wurde die Temperatur auf 155 °C erhöht und kontinuierlich 100 g Hexenoxid (entsprechend 1,0 mol) in den Reaktor gegeben. Bei Erreichen der Druckkonstanz wurde die Temperatur zur Vervollständigung des Umsatzes zwei Stunden gehalten. Man erhielt 485 g der Substanz des Beispiels 24.

Die Substanz des Beispiels 25 wurde analog hergestellt.

### Beispiel 25: i-Decanol + 10 EO + 1,2 Hexenoxid

In den Beispielen 1 bis 25 steht Pentenoxid für 1,2-Pentenoxid, Hexenoxid steht für 1,2-Hexenoxid und Butylenoxid steht für 1,2-Butylenoxid. Die Abkürzung EO steht für Ethylenoxid, PO steht für Propylenoxid und BuO für 1,2-Butylenoxid.

In einzelnen der Beispiele 1 bis 25 werden Alkoholgemische in der Alkoxylierungsreaktion eingesetzt, deren Zusammensetzung wie folgt zu beschreiben ist:
i-Decanol steht für das Isomerengemisch verzweigter C₁₀-Alkanole, welches bei der Trimerisierung von Propylen und nachfolgender Hydroformylierung entsteht.

C_{13/15}-Oxoalkohol steht für das Gemisch aus isomeren C₁₃- und C₁₅-Alkanolen, welches bei der Hydroformylierung von Gemischen der entsprechenden linearen 1-Alkene, also α-Dodecen und α-Tetradecen, entsteht. Die so enthaltenen C₁₃- und C₁₅-Alkanole sind linear oder weisen maximal eine Verzweigung auf.

2-Propylheptanol steht für ein Gemisch von C₁₀-Alkanolen, welches 80 - 100 Gew-% des Isomeren 2-Propylheptanol enthält.

C_{8/10}-Alkohol steht für ein handelsübliches Gemisch aus linearem Octanol und linearem Decanol.

C_{10/12}-Alkohol steht für ein handelsübliches Gemisch aus linearem Decanol und linearem Dodecanol.

C₁₁-Alkohol steht für ein handelsübliches Gemisch aus isomeren C₁₁-Alkanolen, welches zudem als Nebenkomponenten mit einem kumulierten Gehalt von zusammen nicht mehr als 10 Gew.-% entsprechende isomere C₁₀- und C₁₂-Alkanole enthält. Das Gemisch enthält 40 bis 60 Gew.-% lineare Alkanole und 40 bis 60 Gew.-% Alkanole, welche eine Verzweigung aufweisen.

C_{12/15}-Alkohol steht für ein handelsübliches Gemisch aus isomeren C₁₂-, C₁₃-, C₁₄-, und C₁₅-Alkanolen. Das Gemisch enthält 30 bis 50 Gew.-% lineare Alkanole und 50 bis 70 Gew.-% Alkanole, welche eine Verzweigung aufweisen.

Die Beispielsverbindungen wurden einer anwendungstechnischen Beurteilung unterzogen. Dabei wurden die Trübungspunkte, die Oberflächenspannung, die Netzwirkung und die Schaumdämpfung untersucht.

Der Trübungspunkt wurde nach DIN 53 917 in Butyldiglykol bestimmt. Dabei wurde die Temperatur ermittelt, oberhalb derer sich die Lösung trübt und somit als Mischung zweier flüssiger Phasen vorliegt.

Die Oberflächenspannung wurde nach DIN 53 914 bestimmt, indem die Kraft in mN/m gemessen wurde, welche notwendig ist, um eine Platte oder einen horizontal aufgehängten Ring aus der Flüssigkeitsoberfläche herauszuziehen.

Die Netzwirkung (Netzvermögen) wurde in Form des Tauchnetzvermögens nach DIN 53 901 bestimmt. Bei dieser Methode wird ein rundes Gewebeplättchen aus Baumwolle in die wäßrige Tensidlösung eingetaucht. Gemessen wird die Zeit in Sekunden (s) vom Augenblick des Eintauchens bis zum Beginn des Sinkens des Gewebeplättchens. Je kürzer diese Zeit, desto besser ist die Netzwirkung des betreffenden Tensids.

Das Schaumdämpfungsverhalten in der Geschirrspülmaschine wurde im sogenannten "Stufen-Ei-Test" geprüft. Hierbei wird durch magnetische Induktionsmessung in einem handelsüblichen Labor-Instrumentenspülautomaten die Zahl der Umdrehungen (U/min) eines Sprüharms in einem Computer erfaßt und dokumentiert. Durch Schaumbildung, die besonders bei Anwesenheit von Proteinen (Eiweiß) auftritt, wird die Rückstoßkraft verringert, wodurch sich die Umdrehungszahl des Sprüharms vermindert. Die Umdrehungszahl stellt somit ein Maß für die Tauglichkeit von Tensiden in Reinigungsgeräten mit hoher Mechanik dar. Zur Durchführung der Prüfung des Schaumdämpfungsverhaltens wird der Spülflotte neben dem zu prüfenden Tensid eine definierte Menge Ei sowie eine alkalische, tensidfreie Basisreinigerformulierung zugesetzt. Die Testzeit beträgt 30 min, während der die Flotte stufenweise auf zunächst 30 °C, anschließend 40, 50 und zuletzt 60 °C aufgeheizt wird und die Temperatur auf jeder Temperaturstufe 5 min konstant gehalten wird. Auf jeder Temperaturstufe stellt sich innerhalb 5 min ein bestimmter Wert der Umdrehungszahl ein. Die in den Tabellen 1 und 2 angegebene Schaumdämpfung ergibt sich als Mittelwert der vier auf den unterschiedlichen Temperaturstufen erhaltenen Umdrehungszahlen.

Die anwendungstechnischen Daten sind in den nachfolgenden Tabellen zusammengefaßt.

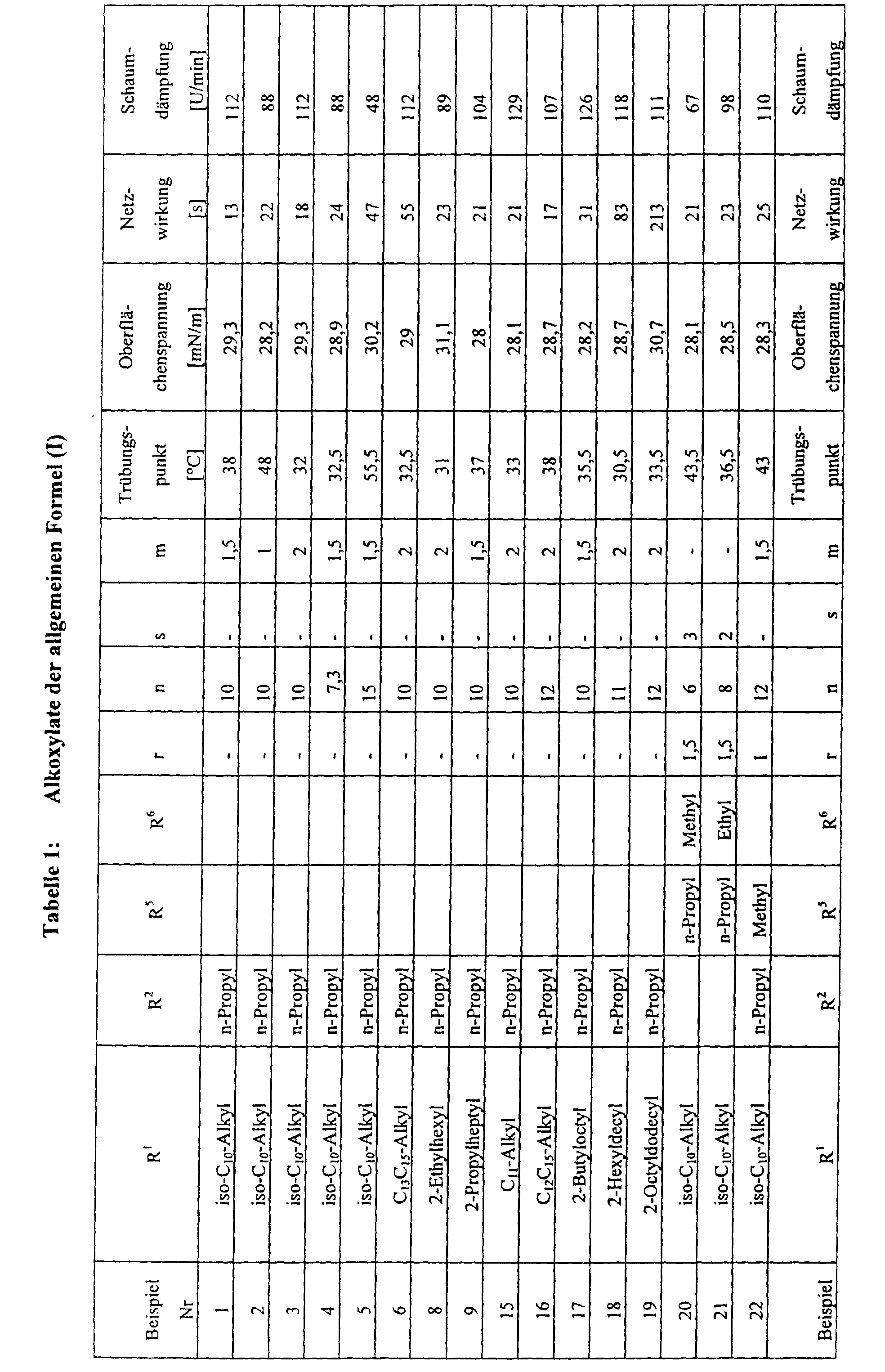

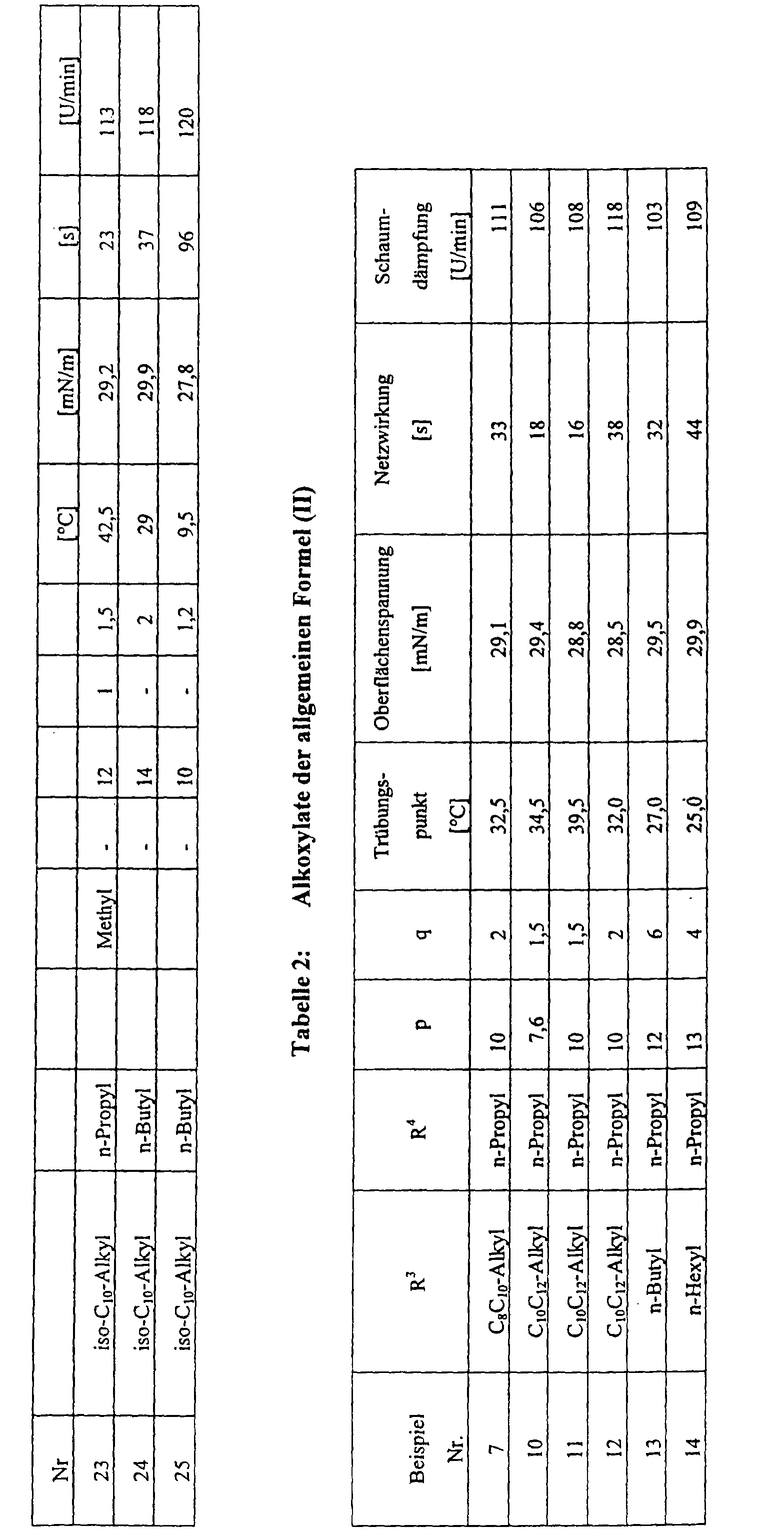

## Patentansprüche

1. Alkoholalkoxylate der allgemeinen Formel (I)
R¹-O-(CH₂-CHR⁵-O-)ᵣ(CH₂-CH₂-O-)ₙ(CH₂-CHR⁶-O-)ₛ(CH₂-CHR²-O-)ₘH (I)
mit der Bedeutung
R¹ mindestens einfach verzweigtes C₄₋₂₂-Alkyl,
R² C₃₋₄-Alkyl
R⁵ C₁₋₄-Alkyl
R⁶ Methyl oder Ethyl
n mittlerer Wert von 1 bis 50
m mittlerer Wert von 0 bis 20
r mittlerer Wert von 0 bis 50
s mittlerer Wert von 0 bis 50,
wobei m mindestens 0,5 ist, wenn R⁵ Methyl oder Ethyl ist oder r den Wert 0 hat,
oder Gemisch aus 20 bis 95 Gew.-% mindestens eines Alkoholalkoxylates der allgemeinen Formel (I) und 5 bis 80 Gew.-% eines entsprechenden Alkoholalkoxylats, in dem R¹ jedoch ein unverzweigter Alkylrest mit gleicher Kohlenstoffzahl ist.

2. Alkoholalkoxylate oder Gemisch nach Anspruch 1, **dadurch gekennzeichnet, daß** R⁵ Methyl ist.

3. Alkoholalkoxylate oder Gemisch nach Anspruch 1 oder 2, **dadurch gekennzeichnet, daß** n einen mittleren Wert von 4 bis 15 aufweist.

4. Alkoholalkoxylate oder Gemisch nach einem der Ansprüche 1 bis 3, **dadurch gekennzeichnet, daß** m einen mittleren Wert von 0,5 bis 4 aufweist.

5. Alkoholalkoxylat der allgemeinen Formel (II)
R³-O-(CH₂-CH₂-O-)ₚ(CH₂-CHR⁴-O-)_{q}H (II)
mit der Bedeutung
R³ verzweigtes oder unverzweigtes C₄₋₂₂-Alkyl
R⁴ C₃₋₄-Alkyl
p mittlerer Wert von 1 bis 50
q mittlerer Wert von 0,5 bis 20,
oder Gemisch aus 5 bis 95 Gew.-% mindestens eines Alkoholalkoxylates der allgemeinen Formel (II), in dem R³ ein verzweigter Alkylrest ist, und 5 bis 95 Gew.-% eines entsprechenden Alkoholalkoxylates, in dem R³ jedoch ein unverzweigter Alkylrest mit gleicher Kohlenstoffzahl ist.

6. Alkoholalkoxylat nach Anspruch 5, **dadurch gekennzeichnet, daß** R³ verzweigtes oder unverzweigtes C₈₋₁₅-Alkyl und R⁴ Propyl bedeuten und q einen mittleren Wert von 0,5 bis 4 aufweist.

7. Verfahren zur Herstellung von Alkoholalkoxylaten oder Gemischen nach einem der Ansprüche 1 bis 6 durch Umsetzung von Alkoholen der allgemeinen Formel R¹-OH bzw. R³-OH oder Gemischen entsprechender verzweigter und unverzweigter Alkohole, gegebenenfalls mit C₃₋₆ Alkylenoxid, sodann mit Ethylenoxid und nachfolgend gegebenenfalls mit C₃₋₄-Alkylenoxid und sodann mit C₅₋₆-Alkylenoxid.

8. Verwendung von Alkoholalkoxylaten oder Gemischen gemäß einem der Ansprüche 1 bis 6 als schaumarme oder schaumdämpfende Tenside.

9. Verwendung nach Anspruch 8 als Tenside in Wasch- und ReinigungsmittelFormulierungen, als Tenside in der metallverarbeitenden Industrie, als Tenside bei der Herstellung und Verarbeitung von Textilien, als Tenside in der Leder-, Papier-, Druck-, Galvano- und Photoindustrie, als Tenside bei der Wasserbehandlung, als Tenside in Pflanzenschutz-Formulierungen, oder als Tenside oder Emulgatoren in der kunststoffherstellenden und kunststoffverarbeitenden Industrie.

10. Wasch- und Reinigungsmittel, enthaltend 0,1 bis 40 Gew.-%, bezogen auf das Gesamtgewicht der Formulierung, mindestens eines Alkoholalkoxylats oder Gemisches gemäß einem der Ansprüche 1 bis 6.

## Claims

1. An alcohol alkoxylate of the formula (I)
R¹-O-(CH₂-CHR⁵-O-)ᵣ(CH₂-CH₂-O-)ₙ(CH₂-CHR⁶-O-)ₛ(CH₂-CHR²-O-)ₘH (I)
where
R¹ is an at least singly branched C₄₋₂₂-alkyl,
R² is C₃₋₄-alkyl
R⁵ is C₁₋₄-alkyl
R⁶ is methyl or ethyl
n is an average value from 1 to 50
m is an average value from 0 to 20
r is an average value from 0 to 50
s is an average value from 0 to 50,
where m is at least 0.5 if R⁵ is methyl or ethyl or r is 0,
or a mixture comprising 20 to 95% by weight of at least one alcohol alkoxylate of the formula (I) and 5 to 80% by weight of a corresponding alcohol alkoxylate but in which R¹ is an unbranched alkyl radical with the same number of carbon atoms.

2. An alcohol alkoxylate as claimed in claim 1, wherein R⁵ is methyl.

3. An alcohol alkoxylate as claimed in claim 1 or 2, wherein n has an average value of from 4 to 15.

4. An alcohol alkoxylate or mixture as claimed in any of claims 1 to 3, wherein m has an average value of from 0.5 to 4.

5. An alcohol alkoxylate of the formula (II)
R³-O-(CH₂-CH₂-O-)ₚ(CH₂-CHR⁴-O-)_{q}H (II)
where
R³ is branched or unbranched C₄₋₂₂-alkyl
R⁴ is C₃₋₄-alkyl
p is an average value of from 1 to 50
q is an average value of from 0.5 to 20,
or a mixture comprising 5 to 95% by weight of at least one alcohol alkoxylate of the formula (I) in which R³ is a branched alkyl radical, and 5 to 95% by weight of a corresponding alcohol alkoxylate but in which R³ is an unbranched alkyl radical with the same number of carbon atoms.

6. An alcohol alkoxylate as claimed in claim 5, wherein R³ is branched or unbranched C₈₋₁₅-alkyl and R⁴ is propyl, and q has an average value of from 0.5 to 4.

7. A process for the preparation of alcohol alkoxylates or mixtures as claimed in any of claims 1 to 6 by reacting alcohols of the formula R¹-OH or R³-OH or mixtures of corresponding branched and unbranched alcohols, optionally with C₃₋₆-alkylene oxide, then with ethylene oxide and subsequently optionally with C₃₋₄-alkylene oxide and then with C₅₋₆-alkylene oxide.

8. The use of alcohol alkoxylates or mixtures as claimed in any of claims 1 to 6 as low-foam or foam-suppressing surfactants.

9. The use as claimed in claim 8 as surfactants in the metal-processing industry, as surfactants in the manufacture and processing of textiles, as surfactants in the leather, paper, printing, electroplating and photographic industry, as surfactants in water treatment, as surfactants in crop-protection formulations, or as as surfactants or emulsifiers in the polymer-production and polymer-processing industry.

10. A detergent or cleaner comprising 0.1 to 40% by weight, based on the total weight of the formulation, of at least one alcohol alkoxylate or mixture as claimed in any of claims 1 to 6.

## Revendications

1. Alcoxylates d'alcool répondant à la formule générale (I) :
R¹-O-(CH₂-CHR⁵-O-)ᵣ(CH₂-CH₂-O)ₙ(CH₂-CHR⁶-O-)ₛ(CH₂-CHR²-O-)ₘH (I)
dans laquelle
R¹ = de l'alkyle en C₄₋C₂₂ ramifié au moins une fois,
R² = de l'alkyle en C₃-C₄,
R⁵ = de l'alkyle en C₁₋C₄,
R⁶ = du méthyle ou de l'éthyle,
n = une valeur moyenne de 1 à 50,
m = une valeur moyenne de 0 à 20,
r = une valeur moyenne de 0 à 50,
s = une valeur moyenne de 0 à 50,
m étant au moins 0,5 lorsque R⁵ est du méthyle ou de l'éthyle ou que r a la valeur de 0,
ou mélange de 20 à 95% en poids d'au moins un alcoxylate d'alcool de formule générale (I) et de 5 à 80% en poids d'un alcoxylate d'alcool correspondant, dans lequel toutefois R¹ est un radical alkyle non ramifié présentant un même nombre de carbones.

2. Alcoxylates d'alcool ou mélange suivant la revendication 1, **caractérisés en ce que** R⁵ est du méthyle.

3. Alcoxylates d'alcool ou mélange suivant l'une des revendications 1 et 2, **caractérisés en ce que** n présente une valeur moyenne de4à15.

4. Alcoxylates d'alcool ou mélange suivant l'une des revendications 1 à 3, **caractérisés en ce que** m présente une valeur moyenne de 0,5 à 4.

5. Alcoxylates d'alcool répondant à la formule générale (II) :
R³-O-(CH₂-CH₂-O-)ₚ(CH₂-CHR⁴-O-)_{q}H (II)
dans laquelle
R³ = de l'alkyle en C₄-C₂₂ ramifié ou non ramifié,
R⁴ = de l'alkyle en C₃-C₄,
p = une valeur moyenne de 1 à 50,
q = une valeur moyenne de 0,5 à 20,
ou un mélange de 5 à 95% en poids d'au moins un alcoxylate d'alcool de la formule générale (II), dans laquelle R³ est un radical alkyle ramifié, et de 5 à 95% en poids d'un alcoxylate d'alcool correspondant, dans lequel R³ toutefois est un radical alkyle non ramifié présentant un même nombre de carbones.

6. Alcoxylate d'alcool suivant la revendication 5, **caractérisé en ce que** R³ représente de l'alkyle en C₈-C₁₅ ramifié ou non ramifié et R⁴ du propyle et **en ce que** q présente une valeur moyenne de 0,5 à 4.

7. Procédé de préparation d'alcoxylates d'alcool ou de mélanges suivant l'une des revendications 1 à 6, par réaction d'alcools de la formule générale R¹-OH ou R³-OH ou de mélanges d'alcools ramifiés et non ramifiés correspondants, éventuellement avec de l'oxyde d'alkylène en C₃-C₆, ensuite avec de l'oxyde d'éthylène et ultérieurement éventuellement avec de l'oxyde d'alkylène en C₃-C₄ et ensuite avec de l'oxyde d'alkylène en C₅-C₆.

8. Utilisation d'alcoxylates d'alcool ou de mélanges suivant l'une des revendications 1 à 6, comme agents tensioactifs pauvres en mousse ou antimousses.

9. Utilisation suivant la revendication 8, comme agents tensioactifs dans des formulations de produits de lavage et de nettoyage, comme agents tensioactifs dans l'industrie de traitement des métaux, comme agents tensioactifs lors de la fabrication et du traitement de matières textiles, comme agents tensioactifs dans l'industrie du cuir, du papier, de l'impression et de la galvano-industrie et de la photo-industrie, comme agents tensioactifs pour le traitement de l'eau, comme agents tensioactifs dans des formulations pour la protection des végétaux, ou comme agents tensioactifs ou émulsionnants dans l'industrie de la fabrication et du traitement des matières synthétiques.

10. Produit de lavage et de nettoyage, contenant 0,1 à 40% en poids, par rapport au poids global de la formulation, d'au moins un alcoxylate d'alcool ou mélange suivant l'une des revendications 1 à 6.
